# EUROPEAN PATENT SPECIFICATION

(11) **EP 0 937 412 B1**
(45) Date of publication and mention of the grant of the patent: **01.02.2006**
(21) Application number: 99103239.2
(22) Date of filing: 19.02.1999
(51) Int. Cl.: A23L 1/275

(54) **Preparation of a finely divided pulverous carotenoid preparation**
Herstellung eines fein verteilten, pulverförmigen Carotinoidpräparats
Production d'une composition finement divisée qui contient des caroténoides

(30) Priority: 23.02.1998 EP 98103113
(43) Date of publication of application: 25.08.1999
(73) Proprietor: DSM IP Assets B.V., 6411 TE Heerlen (NL)
(72) Inventor: Stein, Hermann, 4410 Liestal (CH); Viardot, Klaus, 4125 Riehen (CH); Yang, Bin, 4313 Möhlin (CH)
(74) Representative: Schwander, Kuno

(56) References cited:
- DE-B- 1 211 911
- US-A- 3 790 688
- US-A- 3 998 753
- US-A- 4 522 743
- US-A- 4 726 955
- US-A- 4 844 934
- US-A- 5 364 563

## Description

The present invention relates to a continuous process for converting carotenoids, retinoids or natural colourants into finely divided pulverous forms which are in particular required for colouring foodstuff and animal feeds.

Various processes have been described to prepare a powder containing the active ingredients with a crystallite size less than 1 µm. Most of the processes are well suited to batch processing applications.

For example, US Patent 3,998,753 describes a batch process for the preparation of a water dispersible carotenoid containing powder, wherein the carotenoid has a particle size of less than 1 µm, which process comprises (a) forming a solution of a carotenoid and an antioxidant in a volatile solvent, said solvent being selected from the group consisting of halogenated aliphatic hydrocarbons such as chloroform, carbon tetrachloride and methylene chloride; (b) forming an aqueous solution of sodium lauryl sulfate, a water soluble carrier composition such as e.g. gelatin, a preservative and a stabilizer, and adjusting said solution to a pH of about 10 to 11 and (c) forming an emulsion of the solutions of steps (a) and (b) by mixing at a high speed and high shear; removing the organic solvent and spray drying the resulting emulsion to obtain a carotenoid powder.

In the European Patent Publication EP-0065193 B1 or the corresponding US Patent 4,522,743 a continuous process for the preparation of finely divided carotenoid and retinoid powders is described, in which the carotenoid or retinoid has a particle size essentially below 0.5 µm. The carotenoid or retinoid is dissolved in a volatile, water-miscible organic solvent within less than 10 seconds at 50-200°C. The carotenoid or retinoid is immediately precipitated in colloidally dispersed form from the resulting molecularly dispersed solution by rapid mixing with an aqueous solution of a swellable colloid at 0-50°C. The preparation of the carotenoid solution and the precipitation of the carotenoid are effected continuously in two mixing chambers. The resulting dispersion is freed of solvent and the dispersing medium in a conventional manner. However, for economical and ecological reasons this process has the disadvantage that a large amount of solvent must be used.

US Patent 3,790,688 describes the production of a water-dispersible β-carotene preparation involving the slow addition of an ethereal or ethanolic solution of β-carotene to a hot solution of a soluble starch with rapid stirring, whereby vegetable oil and/or other solvents, exemplified by acetone alone, can also be included to dissolve the β-carotene, heating the resulting mixture to 100-121°C, and, where a powdered preparation is finally desired, precipitating this by adding methanol or ethanol to a concentration of 50-70% to the aforementioned, presumably cooled, mixture. "Soluble starch" is not explained in the US patent, so that the involvement of swellable colloids, e.g. a starch or starch derivative if swellable, is not clearly indicated.

US Patent 4,844,934 describes the preparation of a water-dispersible carotenoid formulation using (vegetable) oil as the "carrier" for dispersing the carotenoid at elevated temperature in the initial process step. This is followed by fine milling, seemingly an essential process step for achieving dissolution, briefly heating the resulting carotenoid solution, introducing the resulting saturated carotenoid solution into an aqueous solution of a three-component protective colloid, e.g. including a starch product such as dextrin, for (rapid) emulsification at an undisclosed temperature, and, if desired, water removal by for example spray drying, and final conversion to a dry powder. The Examples disclose to some extent temperatures and durations of the operations not disclosed in the general description, from which it is clear in particular that a rapid conversion of the carotenoid dispersion to the desired saturated solution in the carrier oil, despite the phrase "for a short time", is not contemplated. The Examples state merely that the β-carotene dispersion is "warmed ... until completely dissolved". The description and Examples provide no clear indication of the temperature at which the subsequent emulsification is performed, but since in the Examples the solution of β-carotene in oil at 180°C is then passed into the aqueous (protective colloid) phase a temperature approaching 180°C seems to be indicated.

US Patent 5,364,563 is a further document in which the described process for producing a powdered carotenoid preparation involves as the initial suspension carrier for the carotenoid a high-boiling oil. It does not indicate the use of a heat exchanger for promoting the rapid dissolution of the carotenoid in the oil; indeed, a "suitable mixer, e.g. an inline mixer" (and superheated steam) is employed. Prior to the dissolution step with superheated steam at about 180°C to about 230°C, the suspension in oil may be milled for reducing the size of any overly course suspended particles, asexemplified in Example 1 and the analogous Example 2, and thereafter the solution is emulsified in an aqueous solution or matrix of a protective colloid and the emulsion is sprayed, e.g. into a bed of starch, and finally dried to a "powder". This powder is actually composed of the colloid having dispersed therein a solution of the carotenoid dissolved in the high-boiling oil. As a consequence, the content of the carotenoid exemplified, i.e. β-carotene, in the powders produced according to the Examples ranges from 6.7% to 7.5% by weight. The powder inevitably contains oil.

US Patent 4,726,955 contains a similar disclosure to the earlier published and above-reviewed EP-0065193 B1/US Patent 4,522,743, and differs essentially in stipulating that the swellable colloid is milk or skimmed milk and that the dissolution temperature of the carotenoid in the volatile, water-miscible organic solvent is 50-240°C rather than 50-200°C.

Finally, German Patent Publication (Auslegeschrift) 1211911 describes a process for producing carotenoid preparations, especially suitable for colouring food and feed products, by emulsifying a solution of a carotenoid in a volatile, water-insoluble organic solvent in an aqueous solution of a swellable protective colloid, and removing the organic solvent from the resulting emulsion in a manner known per se. The organic solvent is said to be preferably a water-insoluble volatile carotenoid solvent, suitably a lower halogenated hydrocarbon, e.g. methylene chloride, or carbon disulphide. The general description provides no information as to the temperatures at which the solution of the carotenoid in the volatile organic solvent is formed, but the Examples clearly indicate that relatively low temperatures are applied, viz. 50°C (Examples 1, 3, 5 and 6), 35°C (Example 2) and 40°C (Example 4). The patent publication further discloses that an advantage of the process is the avoidance of using an oil or a fat as a carotenoid solvent, which in view of the low solubility of the carotenoid in such a solvent would necessitate using high temperatures to achieve the dissolution, but result in undesirable decomposition and isomerization of the carotenoid.

It is an object of the present invention to provide a process that overcomes the aforesaid drawback while converting the active ingredient into finely divided pulverous form.

It has now been found that it is possible to provide a pulverous preparation wherein the active ingredient is finely divided by using a particular water-immiscible organic solvent in a continuous process.

Thus, the present invention relates to a continuous process for the preparation of a pulverous carotenoid, retinoid or natural colourant preparation, wherein the active ingredient is finely divided, which process comprises the steps of
a) forming a suspension of the active ingredient in a water-immiscible organic solvent, being dimethyl carbonate, ethyl formate, ethyl or isopropyl acetate, methyl tert. butyl ether or methylene chloride, optionally containing an antioxidant and/or an oil,
b) feeding the suspension of step a) to a heat exchanger and heating said suspension to 100-180°C, whereby the residence time in the heat exchanger is less than 5 seconds,
c) rapidly mixing the solution of step b) at a temperature in the range of 20-100°C with an aqueous solution of a swellable colloid optionally containing a stabilizer,
d) removing the organic solvent and
e) converting the dispersion of step d) into a pulverous preparation.

The term "finely divided" denotes in the scope of the present invention a particle size of less than 1.5 µm, preferably less than 1 µm, more preferably less than 0.4 µm.

The term "active ingredient" denotes in the scope of the present invention carotenoids, retinoids or natural colourants.

Carotenoids for the purpose of the present invention in particular include beta-carotene, beta-apo-4'-carotenal, beta-apo-8'-carotenal, beta-apo-12'-carotenal, beta-apo-8'-carotenic acid, astaxanthin, canthaxanthin, zeaxanthin cryptoxanthin, citranaxanthin, lutein, lycopene, torularhodin-aldehyde, torularhodin-ethylester, neurosporaxanthin-ethylester, zeta-carotene and dehydroplectaniaxanthin. Also included are carotenoids of natural sources. Preferred are beta-carotene, astaxanthin, canthaxanthin, beta-apo-8'-carotenal and lycopene; more preferred is beta-carotene.

Natural colourants for the purpose of the present invention in particular include curcumine, cochineal, carmine, annatto and mixtures thereof.

Preferably the process of the invention is carried out using carotenoids.

The temperature of step b) is preferably 120-180°C, more preferably 140-170°C, and the temperature of step c) is preferably 50-80°C.

The residence time in the heat exchanger is preferably 0.5-4 seconds, more preferably 1-3 seconds.

The term "water-immiscible organic solvent" denotes an organic solvent having a solubility in water of less than 10% under atmospheric pressure. Such water-inmiscible organic solvents for carrying out the continous process according to the invention are halogenated aliphatic hydrocarbons such as e.g. chloroform, carbon tetrachloride and methylene chloride, water-immiscible esters such as e.g. carbonic acid dimethylester (dimethyl carbonate), formic acid ethylester (ethyl formate) methyl, ethyl or isopropyl acetate; or water-immiscible ethers such as e.g. methylt tert. butyl ether and the like. In the claimed scope of the present invention these are dimethyl carbonate, ethyl formate, ethyl or isopropyl acetate, methyl tert. butyl ether and methylene chloride.

The term "swellable colloids" denotes in the scope of the present invention gelatin, carbohydrates such as e.g. starch or starch derivatives, dextrin, pectin, gum arabic, octenylbutanedioate amylodextrin (CAPSUL™), milk protein such as e.g. casein and vegetable protein as well as mixtures thereof. Preferred are fish gelatin or starch derivatives.

To increase the stability of the carotenoid it is advantageous to add an antioxidant being selected from the group consisting of ascorbic acid, ascorbyl palmitate, dl-alpha-tocopherol, mixed tocopherols, lecithin, butylhydroxytoluol, butyl-4-methoxyphenol and combinations of these compounds.

The antioxidant can be added either to the matrix solution or to the carotenoid solution or to both solutions. A preferred antioxidant for the carotenoid solution is dl-alpha-tocopherol and for the aqueous phase solution it is ascorbyl palmitate.

It may be further advantageous to dissolve an oil in the carotenoid suspension, preferrably corn oil.

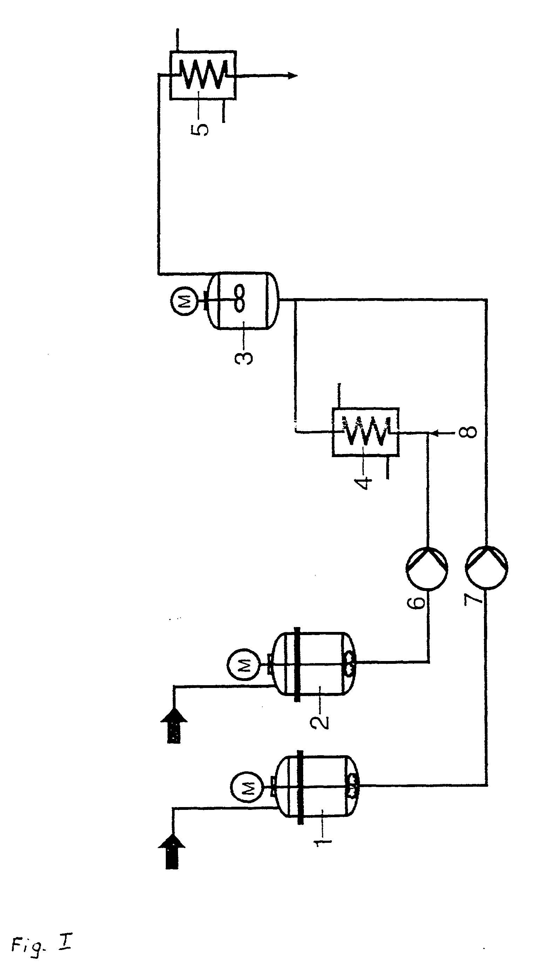

Reference is now made to the accompanying drawing Fig. 1 where a flow chart suitable for carrying out the process in accordance with the instant invention is diagrammatically illustrated. The whole process has to be carried out continously.

The flow chart is explained as follows:
An aqueous matrix containing a swellable colloid and optionally a stabilizer is prepared in Kettle 1.
A suspension of a carotenoid in the selected solvent is prepared in Kettle 2. The suspension may further contain an antioxidant and an oil.

The carotenoid suspension is fed by pump 6 to the heat exchanger 4. The flow rate is adjusted according to the desired residence time which is necessary to dissolve the carotenoid in the solvent at a given temperature. In the heat exchanger 4 the carotenoid suspension is heated to 100 to 180°C, preferably to 120 to 180°C, more preferably to 140 to 170°C, and the carotene is solubilized. The heating can be done either indirectly through the heat exchanger or directly by mixing with steam at 8. The residence time in the heat exchanger is less than 5 seconds, preferably 1 to 3 seconds

The matrix solution of Kettle 1 is fed by pump 7 to Kettle 3. The flow rate depends on the suspension flow rate and the required emulsion composition. In Kettle 3 the carotenoid suspension and the matrix are mixed and emulsified by using a rotor stator homogenizer to the desired particle size of the inner phase of approx. 150-400 nm. As a result of the mixing the temperature is lowered to the range 20 to 100°C.

The dispersion obtained passes to a second heat exchanger 5 whereby the dispersion is cooled. The pressure is released to atmospheric pressure by pressure control.

The solvent is removed using conventional methods e.g. by evaporation. A pulverous composition can be isolated from the resulting dispersion by conventional methods, for example by spray drying or by using powder catch technique.

Using this invention it is possible to manufacture powders which cover very wide range of color.

The manner in which the process of the invention may readily be carried out is illustrated by the following examples. The color intensity was measured in an aqueous dispersion containing 5 ppm carotenoid and given by the calculated extinction of 1% solution in a 1 cm cuvette (E1/1-value). The average particle size has been measured by Coulter Particle Analyzer N4S. The carotenoid content was measured by UV-spectroscopy.

### Example 1

Solvent: ethyl acetate, indirect heat transfer.

The aqueous matrix was prepared in Kettle 1. Thus, 1.0 kg of ascorbyl palmitate was dispersed in 27.8 kg of water at 60°C. The pH-value of this dispersion was adjusted with NaOH (20%) to 7.2-7.6. Then 3.4 kg of fish gelatin and 7.2 kg of sucrose were added. The resulting mixture was stirred until a viscous, clear solution was obtained.

0.75 kg of all-trans-β-carotene cryst. were dispersed in Kettle 2 in a mixture of 90 g of dl-α-tocopherol, 330 g of corn oil and 7.5 kg of ethyl acetate.

The carotene suspension was fed continuously at a rate of 6 kg/h via pump 6 to the heat exchanger 4, heated to 160°C and the carotene was solubilized. The residence time in the heat exchanger was 4 sec.

The matrix solution of Kettle 1 was fed via pump 7 with a flow rate of 9.2 kg/h to Kettle 3 and mixed with the carotene solution.

The resulting emulsion was cooled in a second heat exchanger 5 to 60°C and the pressure was released to atmospheric pressure.

Ethyl acetate was removed in a thin film evaporator. The resulting emulsion showed a particle size of the inner phase of 225 nm and was spray dried. A powder with the following specifications was obtained: 11.6 % carotene content, E ¹/₁ =1015, λ max. 440-460 nm. The powder was well soluble in cold water with an intense red coloration.

### Example 2

Solvent: isopropyl acetate, direct heat transfer (steam) 1.25 kg of Ascorbyl palmitate was dispersed in 30.9 kg water at 60°C according to Example 1. The pH-value of this dispersion was adjusted with NaOH (20%) to 7.2-7.6. Then 5.1 kg of fish gelatin and 7.1 kg of sucrose were added. The resulting mixture was stirred until a viscous, clear solution was obtained.

0.75 kg of Canthaxanthin cryst. were dispersed in Kettle 2 in a mixture of 0.10 kg of dl-α-tocopherol, 0.36 kg of corn oil and 6.25 kg of isopropyl acetate.

The canthaxanthin suspension was fed continuously at a rate of 6 kg/h via pump 6 to the mixing chamber where the temperature was raised by injection of steam to 170°C. Then, the hot canthaxanthin dispersion passed within 2 sec. through the heat exchanger 4 where the canthaxanthin was solubilized.

The matrix solution of Kettle 1 was fed via pump 7 with a flow rate of 8.1 kg/h to Kettle 3 and mixed with the canthaxanthin solution.

The resulting emulsion is cooled in heat exchanger 5 to 60°C and the pressure was released to atmospheric pressure.

Isopropyl acetate was removed in a thin film evaporator. The resulting emulsion showed a particle size of the inner phase of 213 nm and was spray dried. A powder with the following specifications was obtained: 12.3 % canthaxanthin content, E ¹/₁ =905, λ max. 470-485 nm. The powder was well soluble in cold water with an intense cherry-red coloration.

### Example 3

Solvent: isopropyl acetate, direct heat transfer (steam) 10.3 kg of Fish gelatin, 20.6 kg of sugar and 2.78 kg of ascorbyl palmitate were dissolved in 27.56 kg of water in Kettle 1. The pH-value of this matrix was adjusted with NaOH (20%) to 7.2 - 7.6.

6.68 kg of β-Carotene, 0.84 kg of dl-α-tocopherol and 3.34 kg of corn oil were dispersed in 33.4 kg of isopropyl acetate in Kettle 2.

The β-carotene suspension was fed by pump 6 with a flow rate of 25 kg/h to the heat exchanger 4 where it was mixed with steam to reach an outlet temperature of 160°C. The residence time in the heat exchanger 4 was 1.0 sec. The matrix was pumped by pump 7 with a flow rate of 34.5 kg/h to Kettle 3 where the solved β-carotene was mixed with the matrix and emulsified in it. The emulsion was cooled down to 60°C in heat exchanger 5.

Isopropyl acetate was removed from the emulsion by using a vertical evaporator. The resulting emulsion showed a particle size of the inner phase of 220 nm and was spray dried.

The final product had a β-carotene content of 11.3%; E1/1: 1159, λ max. 440-460 nm. The powder was well soluble in water. The solution had a very intensive yellow color.

### Example 4

Solvent: isopropyl acetate, direct heat transfer (steam).

9.25 kg of Fish gelatin, 18.5 kg of sugar and 2.5 kg of ascorbyl palmitate were dissolved in 30.25 kg of water in Kettle 1. The pH-value of this matrix was adjusted with NaOH (20%) to 7.2 -7.6.

6.0 kg of β-Carotene, 0.75 kg of dl-α-tocopherol and 3.0 kg of corn oil were dispersed in 30.0 kg of isopropyl acetate in Kettle 2.

The β-carotene suspension was fed by pump 6 with a flow rate of 20 kg/h to the heat exchanger 4 where it was mixed with steam to reach an outlet temperature of 158°C. The residence time in the heat exchanger 4 was 1.3 sec. The matrix was pumped by pump 7 with a flow rate of 30.4 kg/h to Kettle 3 where the solved β-carotene was mixed with the matrix and emulsified in it. The emulsion is cooled down to 60°C in heat exchanger 5.

Isopropyl acetate was removed from the emulsion by using a vertical evaporator. The resulting emulsion showed a particle size of the inner phase of 240 nm and was spray dried.

The final product has a β-carotene content of 11.2%, E1/1:795, λ max. 440-460 nm. The powder was well soluble in water, the solution has a very intensive red color.

### Example 5

Solvent: methylene chloride, direct heat transfer (steam).

9.25 kg Fish Gelatin, 18.5 kg of sugar and 2.5 kg of Ascorbyl palmitate were dissolved in 30.25 kg of water in Kettle 1. The pH-value of this matrix was adjusted with NaOH (20%) to 7.2 - 7.6.

6.0 kg of β-Carotene, 0.75 of kg dl-α-tocopherol and 3.0 kg of corn oil were dispersed in 30.0 kg of methylene chloride in Kettle 2.

The β-carotene suspension was fed by pump 6 with a flow rate of 20 kg/h to the heat exchanger 4 where it was mixed with steam to reach an outlet temperature of 145°C. The residence time in the heat exchanger 4 was 1.3 sec. The matrix was pumped by pump 7 with a flow rate of 30.4 kg/h to the Kettle 3 where the solved β-carotene was mixed with the matrix and emulsified in it. The emulsion was cooled down to 35°C in heat exchanger 5.

Methylene chloride was removed from the emulsion by using a vertical evaporator. The resulting emulsion showed a particle size of the inner phase of 196 nm and was spray dried.

The final product has a β-carotene content of 9.9%, E1/1: 1120, λₘₐₓ: 440-460 nm. The powder was well soluble in water, the solution has a very intensive yellow color.

## Claims

1. A continuous process for the preparation of a pulverous carotenoid, retinoid or natural colourant preparation, wherein the active ingredient is finely divided, which process comprises the steps of
a) forming a suspension of the active ingredient in a water-immiscible organic solvent, being dimethyl carbonate, ethyl formate, ethyl or isopropyl acetate, methyl tert. butyl ether or methylene chloride, optionally containing an antioxidant and/or an oil,
b) feeding the suspension of step a) to a heat exchanger and heating said suspension to 100-180°C, whereby the residence time in the heat exchanger is less than 5 seconds,
c) rapidly mixing the solution of step b) at a temperature in the range of 20-100°C with an aqueous solution of a swellable colloid optionally containing a stabilizer,
d) removing the organic solvent and
e) converting the dispersion of step d) into a pulverous preparation.

2. A process according to claim 1, wherein the temperature of step b) is 120-180°C.

3. A process according to claim 2, wherein the temperature of step b) is 140-170°C and the temperature of step c) is 50-80°C.

4. A process according to any one of claims 1-3, wherein the residence time in the heat exchanger is 0.5-4 seconds.

5. A process according to claim 4, wherein the residence time in the heat exchanger is 1-3 seconds.

6. A process according to any one of claims 1-5, wherein the active ingredient is a carotenoid.

7. A process according to claim 6, wherein the carotenoid is selected from the group consisting of beta-carotene, beta-apo-4'-carotenal, beta-apo-8'-carotenal, beta-apo-12'-carotenal, beta-apo-8'-carotenic acid, astaxanthin, canthaxanthin, zeaxanthin cryptoxanthin, citranaxanthin, lutein, lycopene, torularhodin-aldehyde, torularhodin-ethylester, neurosporaxanthin-ethylester, zeta-carotene and dehydroplectaniaxanthin.

8. A process according to any one of claims 1-7, wherein the swellable colloid is selected from the group consisting of gelatin, starch, starch derivatives, dextrin, pectin, gum arabic, octenylbutanedioate amylodextrin, milk protein, vegetable protein as well as mixtures thereof.

9. A process according to any one of claims 1-8, wherein the antioxidant is selected from the group consisting of ascorbic acid, ascorbyl palmitate, dl-alpha-tocopherol, mixed tocopherols, lecithin, butylhydroxytoluol, butyl-4-methoxy-phenol and combinations of these compounds.

10. A process according to any one of claims 1-9, wherein the solution of the active ingredient is effected either indirectly through the heat exchanger or directly by mixing with steam and the precipitation of the active ingredient in the swellable colloid is effected continuously in a mixing device connected in series.

11. A pulverous caroteneoid, retioned or natural colourant preparation preparable by a process as claimed according to any one of claims 1-10 and containing from 0.5-25% by weight of the carotenoid, retinoid or natural colourant active ingredient, the particle size of the finely divided active ingredient being less than 1.5 µm.

## Patentansprüche

1. Kontinuierliches Verfahren zur Herstellung eines pulverförmigen Carotinoid-, Retinoid- oder Naturfarbstoffpräparats, in dem der Wirkstoff fein verteilt ist, wobei das Verfahren die Schritte:
a) Bilden einer Suspension des Wirkstoffs in einem nicht mit Wasser mischbaren organischen Lösungsmittel, das Dimethylcarbonat, Ethylformiat, Ethyl- oder Isopropylacetat, Methyl-tert-butylether oder Methylenchlorid ist, gegebenenfalls enthaltend ein Antioxidationsmittel und/oder ein Öl;
b) Einspeisen der Suspension von Schritt a) in einen Wärmetauscher und Erwärmen der Suspension auf 100 bis 250 °C, wobei die Verweilzeit in dem Wärmetauscher weniger als 5 Sekunden beträgt;
c) schnelles Mischen der Lösung von Schritt b) bei einer Temperatur im Bereich von 20 bis 100 °C mit einer wässerigen Lösung aus einem quellbaren Kolloid, gegebenenfalls enthaltend einen Stabilisator;
d) Entfernen des organischen Lösungsmittels und
e) Umwandeln der Dispersion von Schritt d) zu einem pulverförmigen Präparat umfaßt.

2. Verfahren nach Anspruch 1, wobei die Temperatur von Schritt b) 120 bis 180 °C beträgt.

3. Verfahren nach Anspruch 2, wobei die Temperatur von Schritt b) 140 bis 170 °C beträgt und die Temperatur von Schritt c) 50 bis 80 °C beträgt.

4. Verfahren nach einem der Ansprüche 1 bis 3, wobei die Verweilzeit in dem Wärmetauscher 0,5 bis 4 Sekunden beträgt.

5. Verfahren nach Anspruch 4, wobei die Verweilzeit in dem Wärmetauscher 1 bis 3 Sekunden beträgt.

6. Verfahren nach einem der Ansprüche 1 bis 5, wobei der Wirkstoff ein Carotinoid ist.

7. Verfahren nach Anspruch 6, wobei das Carotinoid aus der Gruppe ausgewählt ist, bestehend aus beta-Carotin, beta-apo-4'-Carotenal, beta-apo-8'-Carotenal, beta-apo-12'-Carotenal, beta-apo-8'-Carotinsäure, Astaxanthin, Canthaxanthin, Zeaxanthincryptoxanthin, Citranaxanthin, Lutein, Lycopin, Torularhodin-aldehyd, Torularhodin-ethylester, Neurosporaxanthin-ethylester, zeta-Carotin oder Dehydroplectaniaxanthin.

8. Verfahren nach einem der Ansprüche 1 bis 7, wobei das quellbare Kolloid aus der Gruppe ausgewählt ist, bestehend aus Gelatine, Stärke, Stärkederivaten, Dextrin, Pektin, Gummi arabicum, Octenylbutandioatamylodextrin, Milchprotein, Pflanzenprotein sowie Gemischen davon.

9. Verfahren nach einem der Ansprüche 1 bis 8, wobei das Antioxidationsmittel aus der Gruppe ausgewählt ist, bestehend aus Ascorbinsäure, Ascorbylpalmitat, dl-alpha-Tocopherol, gemischten Tocopherolen, Lezithin, Butylhydroxytoluol, Butyl-4-methoxy-phenol und Kombinationen von diesen Verbindungen.

10. Verfahren nach einem der Ansprüche 1 bis 9, wobei die Lösung aus dem Wirkstoff entweder indirekt durch den Wärmetauscher oder direkt durch Mischen mit Dampf bewirkt wird, und die Ausfällung des Wirkstoffes in dem quellbaren Kolloid kontinuierlich in einer Mischvorrichtung, die in Reihe geschalten ist, bewirkt wird.

11. Pulverförmiges Carotinoid-, Retinoid oder Naturfarbstoffpräparat, das durch ein Verfahren nach einem der Ansprüche 1 bis 10 herstellbar ist, und 0,5 bis 25 Gew.-% des Carotinoid-, Retinoid- oder Naturfarbstoffwirkstoffes enthält, wobei die Teilchengröße des fein verteilten Wirkstoffes weniger als 1,5 µm beträgt.

## Revendications

1. Procédé continu de préparation d'une composition pulvérisée d'un caroténoïde, d'un rétinoïde ou d'un colorant naturel, dans lequel l'ingrédient actif est finement divisé, lequel procédé comprend les étapes de :
a) formation d'une suspension de l'ingrédient actif dans un solvant organique immiscible dans l'eau, étant le carbonate de diméthyle, le formiate d'éthyle, l'acétate d'éthyle ou d'isopropyle, l'oxyde de méthyle et de tert-butyle ou le chlorure de méthylène, contenant facultativement un antioxydant et/ou une huile,
b) introduction de la suspension de l'étape a) à un échangeur de chaleur et chauffage de ladite suspension à une température de 100 à 250ºC, moyennant quoi le temps de séjour dans l'échangeur de chaleur est inférieur à 5 secondes,
c) mélange rapide de la solution de l'étape b) à une température dans l'intervalle de 20 à 100ºC avec une solution aqueuse d'un colloïde capable de gonfler contenant facultativement un stabilisateur,
d) élimination du solvant organique et
e) conversion de la dispersion de l'étape d) dans une préparation pulvérisée.

2. Procédé selon la revendication 1, dans lequel la température de l'étape b) est de 120 à 180ºC.

3. Procédé selon la revendication 2, dans lequel la température de l'étape b) est de 140 à 170ºC et la température de l'étape c) est de 50 à 80ºC.

4. Procédé selon l'une quelconque des revendications 1 à 3, dans lequel le temps de séjour dans l'échangeur de chaleur est de 0,5 à 4 secondes.

5. Procédé selon la revendication 4, dans lequel le temps de séjour dans l'échangeur de chaleur est de 1 à 3 secondes.

6. Procédé selon l'une quelconque des revendications 1 à 5, dans lequel l'ingrédient actif est un caroténoïde.

7. Procédé selon la revendication 6, dans lequel le caroténoïde est choisi dans le groupe constitué par le β-carotène, le β-apo-4'-caroténal, le β-apo-8'-caroténal, le β-apo-12'-caroténal, l'acide β-apo-8'-caroténique, l'astaxanthine, la canthaxanthine, la zéaxanthine, cryptoxanthine, la citranaxanthine, la lutéine, le lycopène, le torularhodin-aldéhyde, le torularhodin-éthylester, le neurosporaxanthine-éthylester, le ζ-carotène et la déhydroplectaniaxanthine.

8. Procédé selon l'une quelconque des revendications 1 à 7, dans lequel le colloïde capable de gonfler est choisi dans le groupe constitué par la gélatine, l'amidon, des dérivés d'amidon, la dextrine, la pectine, la gomme arabique, l'octénylbutanedioate amylodextrine, une protéine du lait, une protéine végétale ainsi que des mélanges de ceux-ci.

9. Procédé selon l'une quelconque des revendications 1 à 8, dans lequel l'antioxydant est choisi dans le groupe constitué par l'acide ascorbique, le palmitate d'ascorbyle, le di-α tocophérol, des tocophérols mélangés, la lécithine, le butylhydroxytoluol, le butyl-4-méthoxyphénol et des combinaisons de ces composés.

10. Procédé selon l'une quelconque des revendications 1 à 9, dans lequel la solution de l'ingrédient actif est réalisée soit indirectement à travers l'échangeur de chaleur soit directement en la mélangeant avec de la vapeur d'eau et la précipitation de l'ingrédient actif dans le colloïde capable de gonfler est réalisée en continu dans un dispositif de mélange relié en série.

11. Préparation pulvérisée de caroténoïde, rétinoïde ou de colorant naturel qui peut être préparée selon un procédé selon l'une quelconque des revendications 1 à 10 et contenant de 0,5 à 25 % en poids de l'ingrédient actif du caroténoïde, du rétinoïde ou du colorant naturel, la granulométrie de l'ingrédient actif finement divisé étant inférieure à 1,5 µm.
